# EUROPEAN PATENT APPLICATION

(11) **EP 4 597 511 A1**
(43) Date of publication of application: **06.08.2025**
(21) Application number: 23870944.8
(22) Date of filing: 27.09.2023
(51) Int. Cl.: G16H 40/60

(54) **ORAL CAVITY SCAN DATA PROCESSING METHOD AND APPARATUS, AND DEVICE**

(30) Priority: 30.09.2022 CN 202211209434
(71) Applicant: Shining 3D Tech Co., Ltd., Hangzhou, Zhejiang 311258 (CN)
(72) Inventor: JIA, Yongjie, Hangzhou, Zhejiang 311258 (CN); SHAO, Maozhen, Hangzhou, Zhejiang 311258 (CN); ZHANG, Jian, Hangzhou, Zhejiang 311258 (CN)
(74) Representative: Grünecker Patent- und Rechtsanwälte PartG mbB
(86) International application number: PCT/CN2023/122136
(87) International publication number: WO 2024/067717

(57) **Abstract**

The disclosure relates to a method and device for processing oral scanning data, and an apparatus. The method includes: performing a scanning task on a plurality of scan regions in an oral cavity in a preset scanning sequence; obtaining target scanning data of the current scan region when a scanning completion event of the current scan region is monitored; performing a data processing task on the target scanning data when no data processing task is being performed; and performing the scanning task on the next scan region according to the scanning sequence, and determining the next scan region as a new current scan region. The disclosure can shorten data processing time and improve scanning efficiency and data processing efficiency.

## Description

The disclosure claims the priority of Chinese Patent Application No. 2022112094343, filed with the Chinese Patent Office on September 30, 2022 and entitled "Method and device for processing oral scanning data, and apparatus", which is incorporated in its entirety herein by reference.

### Technical Field

The disclosure relates to the technical field of computers, in particular to a method and device for processing oral scanning data, and an apparatus.

### Background

In a current method for processing intra-oral scanning data, a complete order is generated only after all inspection ranges such as the upper jaw, the lower jaw and the entire jaw are completely scanned, and then the scanning data of the multiple inspection ranges in the order are optimized and processed separately. By such a method, long time is consumed to process data, and the efficiency is extremely low.

### Summary

### (I) Technical problem to be solved

The technical problem to be solved by the disclosure is to solve problems of long time consumed to process data and low efficiency of an existing method for processing intra-oral scanning data.

### (II) Technical solutions

In order to solve the above technical problems, examples of the disclosure provide a method and device for processing oral scanning data, and an apparatus.

The disclosure provides a method for processing oral scanning data. The method includes:
performing a scanning task on a plurality of scan regions in an oral cavity in a preset scanning sequence;
obtaining target scanning data of a current scan region when a scanning completion event of the current scan region is monitored;
performing a data processing task on the target scanning data when no data processing task is being performed; and
performing the scanning task on a next scan region in the scanning sequence, and determining the next scan region as a new current scan region.

Alternatively, the method further includes:
in a process of performing the data processing task on the target scanning data, when a scanning start event of the next scan region is monitored, reducing computing resources occupied by the data processing task currently being performed.

Alternatively, the method further includes:
in a process of performing the data processing task on the target scanning data, when a scanning completion event of the next scan region is monitored, performing the data processing task on the target scanning data by using all available computing resources until a new scanning start event is monitored, and reducing computing resources occupied by the data processing task currently being performed.

Alternatively, the reducing computing resources occupied by the data processing task currently being performed includes:
determining a first computing resource to be released from the computing resources occupied by the data processing task currently being performed according to a preset proportion and/or a preset lowest computing resource threshold; and
performing the data processing task on the target scanning data by using remaining computing resources after the first computing resource is released.

Alternatively, the reducing computing resources occupied by the data processing task currently being performed includes:
obtaining a second computing resource required for performing the scanning task and a third computing resource currently available;
determining a fourth computing resource to be allocated to the scanning task from the computing resources occupied by the data processing task currently being performed when the second computing resource is greater than the third computing resource; and
performing the data processing task on the target scanning data by using remaining computing resources after the fourth computing resource is released.

Alternatively, the performing the scanning task on a next scan region includes:
determining the released first computing resource after the computing resources occupied by the data processing task currently being performed are reduced;
determining an available fifth computing resource according to the first computing resource and computing resources currently not occupied; and
performing the scanning task on the next scan region by using the fifth computing resource.

Alternatively, the method further includes:
after scanning of the plurality of scan regions is completed in the scanning sequence, processing, by using all available computing resources, scanning data that are not processed.

Alternatively, the method further includes:
when a rescanning event for a target scan region is monitored, interrupting processing of scanning data of the target scan region, and starting to rescan the target scan region.

The disclosure further provides a device for processing oral scanning data, and an apparatus. The device includes:
a scanning module configured to perform a scanning task on a plurality of scan regions in an oral cavity in a preset scanning sequence;
a data obtaining module configured to obtain target scanning data of a current scan region when a scanning completion event of the current scan region is monitored;
a data processing module configured to perform a data processing task on the target scanning data when no data processing task is being performed; and
a region determining module configured to perform the scanning task on a next scan region in the scanning sequence, and determine the next scan region as a new current scan region.

The disclosure further provides an electronic apparatus. The electronic apparatus includes:
a processor, and
a memory configured to store a processor-executable instruction, where
the processor is configured to read the executable instruction from the memory and execute the instruction to implement the method.

### (III) Beneficial effects

Compared with the related art, the technical solution according to the examples of the disclosure has the advantages as follows:
The examples of the disclosure provide the method and device for processing oral scanning data, and an apparatus. The method includes: performing a scanning task on a plurality of scan regions in an oral cavity in a preset scanning sequence; obtaining target scanning data of the current scan region when a scanning completion event of the current scan region is monitored; performing a data processing task on the target scanning data when no data processing task is being performed; and performing the scanning task on the next scan region in the scanning sequence, and determining the next scan region as a new current scan region. In the technical solution, when scanning of a scan region is completed, a data processing task may be prepared to be performed on scanning data of the scan region, scanning gap time between scan regions is fully used, data processing time is shortened, and scanning efficiency and data processing efficiency are improved.

It should be understood that both the foregoing general description and the following detailed description are illustrative and interpretative merely and do not limit the disclosure.

### Brief Description of the Drawings

The accompanying drawings are incorporated in the description as a constituent part of the description, illustrate examples conforming to the disclosure, and serve to explain the principles of the disclosure together with the specification.

In order to describe the technical solutions in examples of the disclosure or the related art more clearly, the accompanying drawings required for describing the examples or the related art will be briefly described below. Apparently, a person of ordinary skill in the art can also derive other accompanying drawings from these accompanying drawings without creative efforts.
Fig. 1 is a flowchart of a method for processing oral scanning data according to some embodiments of the disclosure;
Fig. 2 is a schematic diagram of a process of processing oral scanning data according to some embodiments of the disclosure;
Fig. 3 is another schematic diagram of a process of processing oral scanning data according to some embodiments of the disclosure;
Fig. 4 is a structural block diagram of a device for processing oral scanning data according to some embodiments of the disclosure; and
Fig. 5 is a schematic structural diagram of an electronic apparatus according to some embodiments of the disclosure.

### Detailed Description of the Embodiments

In order to make the objectives, technical solutions, and advantages of the examples of the disclosure clearer, the technical solutions In some embodiments of the disclosures of the disclosure will be clearly and completely described below. Apparently, the described examples are some examples rather than all examples of the disclosure. On the basis of the examples of the disclosure, all other examples derived by those of ordinary skill in the art without creative efforts fall within the scope of protection of the disclosure.

In a current method for processing oral scanning data, all regions in an oral cavity need to be scanned before data processing is performed. For example, in an actual scene, only after all scan regions such as an upper jaw, a lower jaw and an entire jaw are scanned, scanning data of the scan regions start to be reconstructed separately, and a reconstructed model is displayed on a display. In this case, plenty of scanning gap time generally exists, such as communicating with a patient, viewing data, and adjusting a scanning device, in a process of performing a scanning task on the scan regions. Accordingly, long time is consumed to process data, wait time is long for users, and the efficiency is extremely low by such a processing method.

In view of this, in order to shorten the time to process data, the examples of the disclosure provide a method and device for processing oral scanning data, and an apparatus. The disclosure is applicable to scenarios of oral scanning and data processing. Scanning data can start to be processed after scanning of a first scan region is completed, and then region scanning and data processing can be performed in parallel. For example, after scanning of an upper jaw region is completed, upper jaw scanning data can start to be processed, and then in a process of scanning a lower jaw region, the upper jaw scanning data may have been processed, and a model corresponding to the upper jaw region may be displayed on a display. For ease of understanding, the examples of the disclosure are described below.

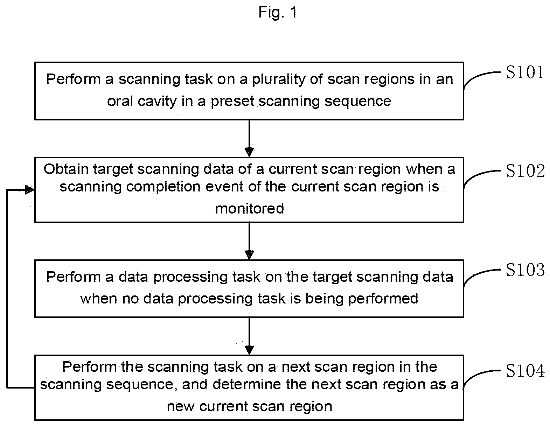

Fig. 1 is a flowchart of a method for processing oral scanning data according to some embodiments of the disclosure. The method may be performed by an apparatus for processing oral scanning data. The apparatus may be implemented by software and/or hardware. With reference to Fig. 1, the method for processing oral scanning data may include the following steps.

S101, a scanning task is performed on a plurality of scan regions in an oral cavity in a preset scanning sequence.

In some embodiments of the disclosure, a scanning sequence of the scan regions such as the upper jaw, the lower jaw, and the entire jaw, in the oral cavity, may be preset. The scanning task may be performed on the scan regions sequentially in the scanning sequence. Alternatively, in some embodiments of the disclosure, the scan regions may be randomly arranged to form a scanning sequence. In actual applications, the scanning sequence may be interrupted, and alternatively, the scanning sequence of the scan regions may be reset as the task progresses. The scanning task may be understood as scanning the scan region from different angles based on a principle of optical scanning technology by scanning apparatuses such as an intraoral scanner, so as to obtain scanning data, such as images and three-dimensional intraoral model data.

S102, target scanning data of a current scan region are obtained when a scanning completion event of the current scan region is monitored.

In some embodiments of the disclosure, the current scan region is a scan region on which the scanning task is currently performed in the scanning sequence. For example, when the scanning task is performed for the first time, the current scan region is a first scan region arranged in the scanning sequence. When the scanning task is completed for the current scan region, a scanning completion event may be triggered, such that target scanning data corresponding to the current scan region is obtained.

S103, a data processing task is performed on the target scanning data when no data processing task is being performed.

When the current scan region is the first scan region, no data processing task is performed, such that after target scanning data of the current scan region are obtained, the data processing task may be performed on the target scanning data. When the current scan region is a non-first scan region, a scan region arranged before the current scan region may have unfinished data processing tasks, that is, data processing tasks being performed. In this case, after the target scanning data of the current scan region are obtained, it may be monitored whether a performed data processing task exists, and when no data processing task is being performed, it indicates that the data processing task has been performed in the last scan region of the current scan region, and thus, the data processing task may be performed on the target scanning data.

The data processing task in some embodiments of the disclosure is generally to perform target classification, disease recognition, fuzzy image elimination, three-dimensional model construction and other processing on the target scanning data.

In some embodiments of the disclosure, not all scan regions need to be scanned before data processing is started, but when scanning of one scan region is completed, the data processing task may be prepared to start being performed on the scanning data of the current scan region. The preparation start refers to that when the data processing task of the previous scan region has not been completed, it is necessary to wait for the completion of the previous data processing task before performance of the data processing task on the scanning data of the current scan region. In some embodiments of the disclosure, the scanning task and the data processing task are performed in parallel, and time and computing resources are reasonably allocated to the scanning task and the data processing task, such that the scanning gap time between the scan regions can be fully used, and wait time of the data processing task can be reduced, such that the data processing time is shortened effectively.

S104, the scanning task is performed on a next scan region in the scanning sequence, and the next scan region is determined as a new current scan region.

After completing the scanning of the current scan region, the scanning task needs to be performed on the next scan region. In some embodiments of the disclosure, a user does not need to select the next scan region to be scanned, but the scanning task is automatically performed on a scan region arranged behind the current scan region in the preset scanning sequence, such that complexity of user operations is reduced. When the scanning task is performed on the next scan region in the scanning sequence, the next scan region is determined as a new current scan region. For example, the current scan region is the upper jaw, after the scanning of the upper jaw is completed, a scan region located one position behind the upper jaw is determined as the lower jaw in the scanning sequence. When the scanning task is performed on the lower jaw, the lower jaw is determined as a new current scan region.

For the new current scan region, S102 is returned to, a scanning completion event is monitored, and the data processing task is performed accordingly.

With respect to S103, in some embodiments of the disclosure, in the process of performing the data processing task on the target scanning data, the computing resources occupied by the data processing task and the scanning task may be allocated and adjusted respectively, so as to guarantee sufficient computing resources for the scan task, avoid the phenomenon of scanning stagnation, and improve the efficiency and smoothness of the scanning task.

In some scenes, in a process of performing the data processing task on the target scanning data, when a scanning start event of the next scan region is monitored, reducing computing resources occupied by the data processing task currently being performed.

During specific implementation, when the data processing task (denoted as Sᵢ) starts to be performed on target scanning data, since the current scan region (denoted as Rᵢ) has been scanned and the next scan region (R_{i + 1}) is not scanned yet, that is, no scanning task is performed, in order to fully use the computing resources and improve the data processing efficiency, all available computing resources may be used to perform the data processing task on target scanning data Sᵢ.

With the progress of the data processing task Sᵢ, when a scanning start event of the next scan region R_{i + 1} is monitored, it indicates that a new scanning task has started to be performed. In this case, in order to guarantee smooth performance of the scanning task, the computing resources occupied by the data processing task Sᵢ currently being performed may be reduced. In this way, some of the computing resources occupied by the data processing task Sᵢ are released, and the released computing resources are scheduled for use by the scanning task R_{i + 1}.

Alternatively, in some other scenes, in a process of performing the data processing task on the target scanning data, when a scanning completion event of the next scan region is monitored, the data processing task is performed on the target scanning data by using all available computing resources until a new scanning start event is monitored, and the computing resources occupied by the data processing task currently being performed are reduced.

During specific implementation, the data processing task often cannot be completed in the scanning gap time between two scan regions, such that the data processing task and the scanning task are performed simultaneously. In this case, a scanning completion event of the next scan region R_{i + 1} (that is, the new current region) may be monitored. When the scanning completion event is monitored, in a case that the data processing task Sᵢ is still not completed, all available computing resources are used to perform the data processing task on the target scanning data Sᵢ. In a process of performing the data processing task by using all available computing resources, a scanning start event of the next scan region (R _{i + 2}) may be monitored until the scanning start event is monitored, and the computing resources occupied by the data processing task Sᵢ currently being performed are reduced.

Alternatively, in some embodiments of the disclosure, the data processing task Sᵢ may be completed before the scanning completion event of the scan region R_{i + 1} is monitored. In this case, the scanning task continues to be performed. With reference to the foregoing example, when the scanning completion event of the scan region R_{i + 1} is monitored, the data processing task is performed on the target scanning data S_{i + 1}.

In the above example, reference may be made to the following implementations for a method for reducing the computing resources occupied by the data processing task currently being performed.

Implementation 1 A first computing resource to be released is determined from the computing resources occupied by the data processing task currently being performed according to a preset proportion and/or a preset lowest computing resource threshold. The data processing task is performed on the target scanning data by using remaining computing resources after the first computing resource is released.

Alternatively, the first computing resource to be released may be determined from the computing resources occupied by the data processing task currently being performed according to the preset proportion (for example, 30%). The first computing resource is 30% of the computing resources occupied by the data processing task currently being performed. After the first computing resource is released, the remaining 70% computing resources are used to perform the data processing task on the target scanning data.

Alternatively, some of computing resources higher than the lowest computing resource threshold may be determined from computing resources occupied by the data processing task currently being performed according to the lowest computing resource threshold. The some of computing resources higher than the lowest computing resource threshold may be used as the first computing resource to be released. The data processing task may be performed on the target scanning data by using the lowest computing resource threshold.

Alternatively, the first computing resource may also be determined according to both the proportion and the lowest computing resource threshold. For example, the first computing resource is determined preferentially according to the proportion, and when the remaining 70% computing resources does not reach the lowest computing resource threshold, the first computing resource is re-determined according to the lowest computing resource threshold.

Correspondingly, the released first computing resource is determined after the computing resources occupied by the data processing task currently being performed are reduced. An available fifth computing resource is determined according to the first computing resource and computing resources currently not occupied. The scanning task is performed on the next scan region by using the fifth computing resource.

Implementation 2 First, a second computing resource required for performing the scanning task and a third computing resource currently available are obtained. The second computing resource required for performing the scanning task may be the minimum computing resource capable of guaranteeing smooth and fluent performance of the scanning task. The third computing resource currently available are available computing resources of the system currently not occupied.

When the second computing resource is greater than the third computing resource, it indicates that the third computing resource currently available cannot support performance of the scanning task. Based on this, a fourth computing resource to be allocated to the scanning task may be determined from computing resources occupied by the data processing task currently being performed. The fourth computing resource is not less than a difference between the second computing resource and the third computing resource. Then, the data processing task is performed on the target scanning data by using remaining computing resources after the fourth computing resource is released.

Accordingly, the scanning task is performed on the next scan region by using the second computing resource or using a sum of the third computing resource and the fourth computing resource.

Moreover, it can be understood that after scanning of the plurality of scan regions is completed in the scanning sequence, the scanning task ends. In order to improve the processing efficiency of the data processing task, in some embodiments of the disclosure, all available computing resources may be used to process the scanning data that are not processed.

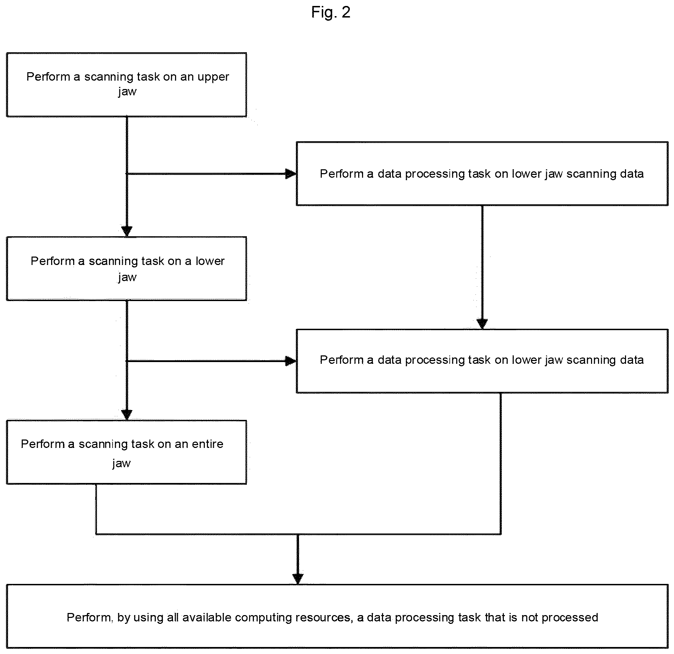

According to the above example, three scan regions of the upper jaw, the lower jaw and the entire jaw inside the oral cavity are taken as some embodiments to provide a process of processing oral scanning data, as shown in Fig. 2.

In some embodiments of the disclosure, the scanning task is started for the upper jaw in the scanning sequence of the upper jaw, the lower jaw and the entire jaw from first to last.

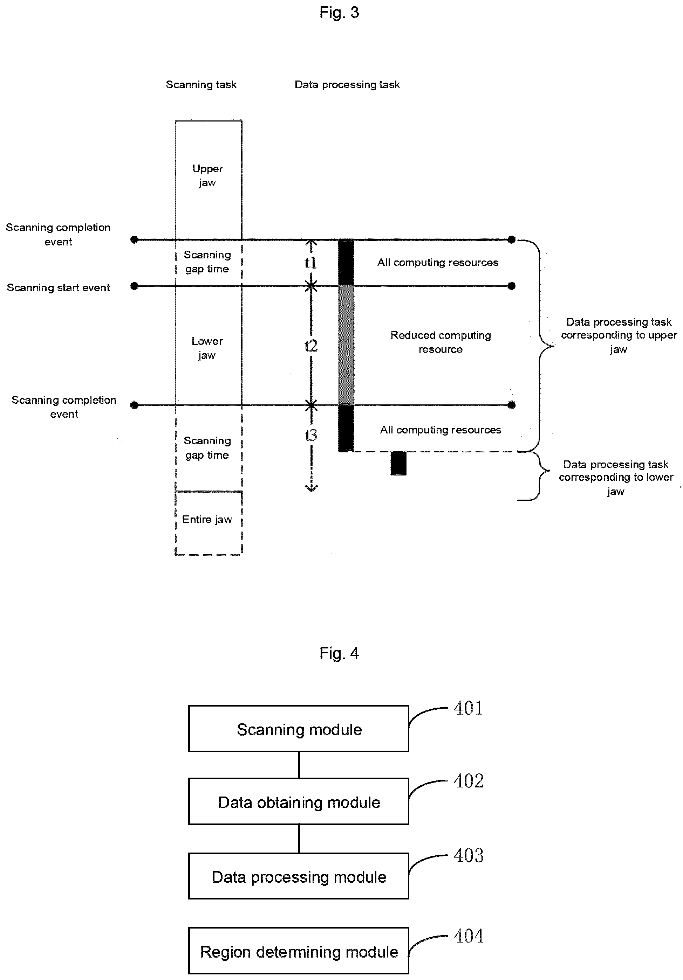

When a scanning completion event of the upper jaw is monitored, scanning data of the upper jaw are obtained. A data processing task is performed on the scanning data of the upper jaw. In a process of performing the data processing task on the scanning data of the upper jaw, in a case where the scanning start event of the lower jaw is not monitored, the data processing task is performed by using all available computing resources within a time period t1 shown in Fig. 3.

When the scanning start event of the lower jaw is monitored, the computing resources occupied by the data processing task currently being performed for the upper jaw are reduced in a time period t2 as shown in Fig. 3, so as to guarantee normal performance of the scanning task of the lower jaw.

Assuming that performance time of the data processing task corresponding to the upper jaw is long, in this scene, when a scanning completion event of the lower jaw is monitored, all available computing resources are used to perform the data processing task corresponding to the upper jaw within a time period t3 as shown in Fig. 3.

When the performance of the data processing task corresponding to the upper jaw is completed, since the scanning of the lower jaw is completed, based on this, the data processing task may start to be performed on the scanning data of the lower jaw.

By analogy, the scanning task and the data processing task are sequentially performed on the scan regions in the scanning sequence. Under the condition that the scanning task and the data processing task are simultaneously performed, the computing resources occupied by the data processing task are reduced, and sufficient computing resources are used to guarantee normal performance of the scanning task. After scanning of the last scan region of the entire jaw is completed, all available computing resources are used to perform the data processing task that are not completed.

In some embodiments of the disclosure, the method for processing oral scanning data may further include: when a rescanning event for a target scan region is monitored, processing of scanning data of the target scan region is interrupted, and the target scan region starts to be rescanned.

In practical applications, in a case that quality of the target scanning data of a scan region is poor (such as missing data, unclear image), and quality of a processing result of the target scanning data is poor, scanning supplement or rescanning for the scan region with the above problems are required.

In some embodiments of the disclosure, a rescanning operation may be initiated for the target scan region by a user. A rescanning event is received according to the rescan operation. Processing of the scanning data of the target scan region is interrupted according to the rescanning event. The target scan region starts to be rescanned. Then, the data processing task is re-performed according to the scanning data obtained by rescanning.

In some embodiments of the disclosure, whether to trigger the rescanning event may be determined according to a data processing result obtained by the data processing task. For example, a determination manner may be to determine whether the data processing result contains a preset key part, and if not, the rescanning event is triggered. When it is determined that a rescanning event is triggered, processing of scanning data of the target scan region is interrupted, and the target scan region starts to be rescanned.

In conclusion, the example of the disclosure provides the method for processing oral scanning data. First, a scanning task is performed on a plurality of scan regions in an oral cavity in a preset scanning sequence; then when a scanning completion event of the current scan region is monitored, target scanning data of the current scan region are obtained, and a data processing task is performed on the target scanning data; and the scanning task is performed on the next scan region in the scanning sequence, and the next scan region is determined as a new current scan region.

In the above technical solution, when scanning of a scan region is completed, a data processing task may be performed on scanning data of the scan region, scanning gap time between scan regions is fully used, data processing time is shortened, and scanning efficiency and data processing efficiency are improved. Moreover, the scanning task is automatically performed sequentially for the scan regions in the scanning sequence, such that complexity of user operations can be reduced. Further, when the data processing task and the scanning task are performed simultaneously, sufficient computing resources are guaranteed for performing the scanning task on the scan region by reducing the computing resources occupied by the data processing task currently being performed, such that scanning efficiency and smoothness of scanning are improved. Accordingly, the disclosure can effectively shorten data processing time and improve data processing efficiency.

Fig. 4 is a structural block diagram of an apparatus for processing oral scanning data according to some embodiments of the disclosure. With reference to Fig. 4, the apparatus includes:
a scanning module 401 configured to perform a scanning task on a plurality of scan regions in an oral cavity in a preset scanning sequence;
a data obtaining module 402 configured to obtain target scanning data of a current scan region when a scanning completion event of the current scan region is monitored;
a data processing module 403 configured to perform a data processing task on the target scanning data when no data processing task is being performed; and
a region determining module 404 configured to perform the scanning task on a next scan region in the scanning sequence, and determine the next scan region as a new current scan region.

The apparatus according to the example has the same implementation principles and technical effects as those In some embodiments of the disclosure of the foregoing method. For a concise description, reference may be made to the corresponding contents In some embodiments of the disclosure of the foregoing method for the parts not mentioned in the apparatus example, which will not be repeated herein.

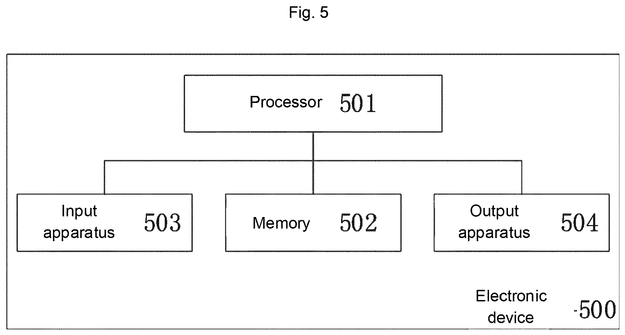

Fig. 5 is a schematic structural diagram of an electronic apparatus according to some embodiments of the disclosure. As shown in Fig. 5, the electronic apparatus 500 includes one or more processors 501 and a memory 502.

The processor 501 may be a central processing unit (CPU) or other forms of processing units having data processing capabilities and/or instruction execution capabilities, and may control other components in the electronic device 500 to perform desired functions.

The memory 502 may include one or more computer program products. The computer program product may include various forms of computer-readable storage media, such as a volatile memory and/or a nonvolatile memory. The volatile memory may include, for example, a random access memory (RAM) and/or a cache. The nonvolatile memory may include, for example, a read only memory (ROM), a hard disk, and a flash memory. One or more computer program instructions may be stored on the computer-readable storage mediums. The program instructions may be executed by the processor 501, so as to implement the method for processing oral scanning data in the embodiments of the disclosure described above and/or other desired functions. Various contents such as input signals, signal components, and noise components may also be stored in the computer-readable storage medium.

In an instance, the electronic device 500 may further include an input device 503 and an output device 504. These components are interconnected by a bus system and/or other forms of connection mechanisms (not shown).

Furthermore, the input device 503 may also include, for example, a keyboard, and a mouse.

The output device 504 may output various information including determined distance information, direction information, and the like to the outside. The output device 504 may include, for example, a display, a speaker, a printer, a communication network, and a remote output apparatus connected to the communication network.

Alternatively, for simplicity, only some of the components related to the disclosure in the electronic device 500 are shown in Fig. 5, and components such as buses, and input/output interfaces are omitted. Moreover, the electronic device 500 may further include any other suitable components according to a specific application.

Further, the embodiments of the disclosure further provide a computer-readable storage medium. The storage medium stores a computer program. The computer program is configured to perform the above method for processing oral scanning data.

A computer program product of a method and device for processing oral scanning data, an electronic device and a medium provided In some embodiments of the disclosures of the disclosure includes a computer-readable storage medium storing program code. Instructions included in the program codes can be used for performing the method described in the foregoing method examples. Reference may be made to the method example for specific implementation, which is not repeated herein.

It should be noted that relation terms herein such as "first" and "second" are merely used to distinguish one entity or operation from another entity or operation without necessarily requiring or implying any such an actual relation or order between these entities or operations. Moreover, the terms "comprise", "include", or their any other variations are intended to cover non-exclusive inclusions. Therefore, a process, method, article, or device including a series of elements not only includes those elements, but also includes other elements that are not explicitly listed, or further includes inherent elements of such a process, method, article, or device. Without more restrictions, the elements defined by the sentence "comprise a ..." or "include a ..." do not exclude the existence of other identical elements in the process, method, article, or device including the elements.

What are described above are merely particular embodiments of the disclosure, through which those skilled in the art can understand or practice the disclosure. Various modifications to these examples will be apparent to those skilled in the art, and the general principles defined herein can be implemented in other examples without departing from the spirit or scope of the disclosure. Thus, the disclosure is to accord with the widest scope consistent with the principles and novel features disclosed herein instead of being limited to the examples described herein.

### Industrial applicability

According to the method for processing oral scanning data provided in the disclosure, when scanning of a scan region is completed, a data processing task may be prepared to be performed on scanning data of the scan region, scanning gap time between scan regions is fully used, data processing time is shortened, scanning efficiency and data processing efficiency are improved, and the method has high industrial practicability.

## Claims

1. A method for processing oral scanning data, comprising:
performing a scanning task on a plurality of scan regions in an oral cavity in a preset scanning sequence;
obtaining target scanning data of a current scan region when a scanning completion event of the current scan region is monitored;
performing a data processing task on the target scanning data when no data processing task is being performed; and
performing the scanning task on a next scan region in the scanning sequence, and determining the next scan region as a new current scan region.

2. The method as claimed in claim 1, wherein the method further comprises:
in a process of performing the data processing task on the target scanning data, when a scanning start event of the next scan region is monitored, reducing computing resources occupied by the data processing task currently being performed.

3. The method as claimed in claim 1, wherein the method further comprises:
in a process of performing the data processing task on the target scanning data, when a scanning completion event of the next scan region is monitored, performing the data processing task on the target scanning data by using all available computing resources until a new scanning start event is monitored, and reducing computing resources occupied by the data processing task currently being performed.

4. The method as claimed in claim 2 or 3, wherein the reducing computing resources occupied by the data processing task currently being performed comprises:
determining a first computing resource to be released from the computing resources occupied by the data processing task currently being performed according to a preset proportion and/or a preset lowest computing resource threshold; and
performing the data processing task on the target scanning data by using remaining computing resources after the first computing resource is released.

5. The method as claimed in claim 2 or 3, wherein the reducing computing resources occupied by the data processing task currently being performed comprises:
obtaining a second computing resource required for performing the scanning task and a third computing resource currently available;
determining a fourth computing resource to be allocated to the scanning task from the computing resources occupied by the data processing task currently being performed when the second computing resource is greater than the third computing resource; and
performing the data processing task on the target scanning data by using remaining computing resources after the fourth computing resource is released.

6. The method as claimed in claim 4, wherein the performing the scanning task on the next scan region comprises:
determining the released first computing resource after the computing resources occupied by the data processing task currently being performed are reduced;
determining an available fifth computing resource according to the first computing resource and computing resources currently not occupied; and
performing the scanning task on the next scan region by using the fifth computing resource.

7. The method as claimed in claim 1, wherein the method further comprises:
after scanning of the plurality of scan regions is completed in the scanning sequence, processing, by using all available computing resources, scanning data that are not processed.

8. The method as claimed in claim 1, wherein the method further comprises:
when a rescanning event for a target scan region is monitored, interrupting processing of scanning data of the target scan region, and starting to rescan the target scan region.

9. A device for processing oral scanning data, comprising:
a scanning module configured to perform a scanning task on a plurality of scan regions in an oral cavity in a preset scanning sequence;
a data obtaining module configured to obtain target scanning data of a current scan region when a scanning completion event of the current scan region is monitored;
a data processing module configured to perform a data processing task on the target scanning data when no data processing task is being performed; and
a region determining module configured to perform the scanning task on a next scan region in the scanning sequence, and determine the next scan region as a new current scan region.

10. An electronic apparatus, comprising:
a processor, and
a memory configured to store a processor-executable instruction, wherein
the processor is configured to read the executable instruction from the memory and execute the instruction to implement the method as claimed in any one of claims 1 to 8.
